Europäisches Patentamt

(19) European Patent Office

Office européen des brevets

(11) Publication number : **0 355 905 B1**

(12) # EUROPEAN PATENT SPECIFICATION

(45) Date of publication of patent specification :
07.01.93 Bulletin 93/01

(51) Int. Cl.⁵ : **A61K 31/725,** C08B 37/10

(21) Application number : 89202061.1

(22) Date of filing : 09.08.89

(54) Heparin fragments and fractions with anti-HIV action.

The file contains technical information submitted after the application was filed and not included in this specification

(30) Priority : 24.08.88 NL 8802087

(43) Date of publication of application :
28.02.90 Bulletin 90/09

(45) Publication of the grant of the patent :
07.01.93 Bulletin 93/01

(84) Designated Contracting States :
AT BE CH DE ES FR GB GR IT LI NL SE

(56) References cited :
EP-A- 0 165 569
EP-A- 0 270 317
CHEMICAL ABSTRACTS, vol. 107, no. 17, 26th October 1987, page 30, abstract no. 146958t, Columbus, Ohio, US; M. ITO et al.:"Inhibitory effect of dextran sulfate and heparin on the replication of human immunodeficiency virus (HIV) in vitro" &ANTIVIRAL RESEARCH, vol. 7, no. 6, 1987, pages 361-367 (Cat. D)

(56) References cited :
PROCEEDINGS OF THE NATIONAL ACADEMY OF SCIENCES, USA, vol. 85, no. 16, August 1988, pages 6132-6136, Washington, US; M. BABAet al.: "Mechanism of inhibitory effect of dextran sulfate and heparin on replication of human immunodeficiency virus in vitro"

(73) Proprietor : **AKZO N.V.**
**Velperweg 76**
**NL-6824 BM Arnhem (NL)**

(72) Inventor : **Ottenheijm, Henricus Carl Joseph**
**Gagelveld 5**
**NL-6596 CC Milsbeek (NL)**
Inventor : **Den Hollander, Franciscus Cornelis**
**Mozartlaan 8**
**NL-5343 EI Oss (NL)**
Inventor : **De Clercq, Erik Desiré Alice**
**Paul Lebrunstraat 35 - bus 7**
**B-3000 Leuven (BE)**

(74) Representative : **Hermans, Franciscus G.M. et al**
**Patent Department AKZO N.V. Pharma Division P.O. Box 20**
**NL-5340 BH Oss (NL)**

EP 0 355 905 B1

## Description

The invention relates to a novel application of heparin fragments and heparin fractions which are obtainable by periodate, heparinase, or nitrite degradation, as a medicament which inhibits or prevents the replication of the human immunodeficiency virus (HIV).

It is known that heparin, but also other mucopolysaccharides, such as dextran sulphate, have in vitro an antiviral effect on the human immunodeficiency virus (HIV). These substances give an appreciable reduction in the cytopathogenicity of HIV for MT-4 and Molt-4 cells. (M. Ito et al., Antiviral Research, 7 (1987), 361-7). Substances of this type could be effective chemotherapeutic agents against HIV and thus could serve to combat AIDS (acquired immune deficiency syndrome) and ARC (AIDS-related complex). The advantage of the said mucopolysaccharides over nucleotides such as 3'-azido-2',3'-dideoxythymidine (AZT, Retrovir[(R)]) is their low cytotoxicity, so that they can serve as selective inhibitors for HIV replication. However, a great drawback which is associated with the use of heparin and dextran sulphate for the prevention of HIV replication is the characteristic (of sulphated glycosaminoglycans) to cause haemorrhaging and/or to interfere with the blood coagulation process, which obviously hamper their practical usefulness as anti-AIDS drugs.

Apart from heparin also fragmented heparins have been tested on HIV-1 replication (M. Baba et al., Proc. Natl. Acad. Sci. USA 85 (1988) 6132-6136). This reference, however, discloses heparin fragments of unknown composition and unknown origin which are less or not active in comparison with nonfragmented heparin.

It has now been found that smaller heparin fragments obtainable by periodate, heparinase, or nitrite degradation, and low-affinity heparin, of which it is known that the risks of haemorrhaging and/or the anti-clotting action are low, retain an appreciable activity as an inhibitor for HIV replication. At low dose they display a strong activity against HIV-1 as well as against HIV-2, and with respect to HIV-2 they are much more active than standard heparin.

It is found that all sorts of heparin fragments display this anti-HIV activity, independently of molecular weight and method of degradation (periodate, heparinase, or nitrite degradation). The criterion on which these substances are chosen is directed towards the haemorrhaging and clotting characteristics. When heparin fragments or heparin fractions are chosen which have an anti-thrombin activity of less than 50 U/mg, and preferably less than 20 U/mg, the risks of haemorrhaging and the anti-clotting action are low. Substances with a minimum anti-thrombin activity of less than 1 U/mg are particularly interesting because the anti-HIV activity has not decreased but the side effects are negligeably small.

The novel product is found to possess no mutagenic and no toxic characteristics, even at high dosages (up to 400 mg/kg/day).

The heparin fraction and fragments can be obtained in various ways. Mammal tissue, such as lungs, pancreas, liver, intestines and, in particular, intestinal mucus, can serve as the raw material. The product is first released from the mammal tissue by autolysis or with the aid of proteolytic enzymes (for example enzymes from the pancreas of pigs, or bacterial enzymes, such as proteases from Bacillus subtilis).

The product is then isolated by precipitation with organic solvents, usually with water-miscible solvents, such as alcohols, for example methanol. Other methods of isolation are based on bonding to a quaternary aliphatic ammonium base or to a basic ion exchanger. Further purification of the product can take place by fractionated precipitation. The heparin can then be separated into a high-affinity and a low-affinity fraction, for example by separation over a AT-III-Sepharose column. The low-affinity fraction can be used as such or can be further degraded.

The high-affinity fraction can be degraded and the fragments thus obtained can then again be separated into high- and low-affinity fractions, the low affinity fraction of which can be used as an anti-HIV medicament.

Degradation can also be carried out with heparin not separated over AT-III. Degraded heparin fragments can be used as such or can still be separated over a AT-III-Sepharose column into a high- and a low-affinity fraction.

A common feature of the heparin fragments and fractions is that they are oligomers or polymers of repeating units which consists of the disaccharides $\alpha$-D-(1 $\rightarrow$ 4)-glucosamine-$\beta$-D-(1 $\rightarrow$ 4)-glucuronic acid and/or $\alpha$-D-(1 $\rightarrow$ 4)-glucosamine-$\alpha$-L-(1 $\rightarrow$ 4)-iduronic acid. The various hydroxy and amino groups can be sulphated. The amino groups can also be acetylated. In the case of fragments, depending on the degradation method chosen, a non-reducing end unit can contain a double bond and/or a reducing glucosamine end can be converted to an anhydromannose.

Degradation methods which can be used are the following methods known in heparin chemistry:
- nitrite degradation
- periodate degradation
- heparinase degradation.

Heparin degraded with periodate and low-affinity heparin degraded with nitrite are particularly suitable because of their favourable therapeutic index.

The heparin degradation with periodate may e.g. be performed by the method as described by Fransson and Lewis (FEBS Lett., 97(1), (1979),119). Preferred are the periodate degraded fragments with a molecular weight of between 2500 and 6500 D.

The action of these heparin fractions and fragments is not known with certainty, but it is assumed that an a-specific interaction occurs between heparin and T-lymphocytes, as a result of which the bond between virus and lymphocyte is inhibited. This would explain why the molecular weight and the specific composition of the heparin fractions or fragments are of minor importance.

The fragments and fractions can be processed to a pharmaceutical dosage form in the manner customary for heparin, for example dissolving in water suitable for injection purposes, to which, if desired, pharmaceutically acceptable auxiliaries (preservatives, certain salts) can also be added. The clinical use is preferably effected by subcutaneous or intravenous (possibly intermittent) injection or by means of infusion. Use as a depot or as a preparation with prolonged release is also very possible. Other means of dosage, such as intra-pulmonary administration via spray inhalation or administration by means of a suppository are also possible.

The substances of this invention can be used in a broad dosage range. Normal dosages are between 0.1 and 400 mg/kg/day; a dosage of between 0.5 and 150 mg/kg/day is preferred.

The invention will be illustrated by the following examples and tests, without these having a restrictive character.

## Example 1

### Periodate treatment

2 g of heparin were dissolved in 40 ml of water and 40 ml of 0.2 M $NaIO_4$ solution were added. The mixture was incubated at room temperature with the exclusion of light for 6 hours at pH 7 and 37 °C. 2 ml of ethylene glycol were then added and the mixture was dialysed with flowing deionized water and then freeze-dried.

1 g of the powder obtained was dissolved in 10 ml of water and the solution was heated to 50 °C. 4 ml of a 0.5 M $NaBH_4$ solution was then added and the mixture was incubated for 2 hours at 50 °C. The pH of the solution was then brought to 3.4 with 2N acetic acid. After 5 minutes, the solution was neutralized to pH 7.1 with 1 N NaOH. The mixture was dialysed with flowing deionized water and freeze-dried.

Molecular weight 6420 (HPLC with respect to heparin standards).

Uronic acid content 90.4% (with respect to heparin).

## Example 2

### Heparinase treatment

Heparin (2% wt/v) was dissolved in 0.25 M ammonium acetate with 0.025 M calcium acetate at pH 5.8 and incubated at 30 °C with heparinase (5 IU/g heparin). At an $OD_{234}$ of 61, a strong basic anionic exchanger was added to the mixture (5 g IW MP500A per g heparin) and after absorption for 2 hours the ion exchanger was eluted after absorption for 2 hours the ion exchanger was eluted with 5 bed volumes of 5% sodium chloride solution. The main fraction was then obtained by elution with 5 bed volumes of 15% sodium chloride solution and precipitation with 4 volumes of 100% methanol. The precipitate was worked up and dried under vacuum.

Molecular weight 2500 (HPLC with respect to heparin standards).

Uronic acid content 98.6% (with respect to heparin).

## Example 3

### Nitrite treatment

Heparin (0.05% wt/v) was dissolved in 0.1 M citric acid buffer at pH 3.0. 1 M sodium nitrite solution was then added (1 ml/10 mg heparin).

After 15 minutes, the mixture was cautiously neutralized with 12.5% (wt/v) ammonium sulphamate (2 ml/10 mg heparin). The mixture was stirred for a further half hour and then left to stand for 1 hour. The solution was concentrated, neutralized with alkali to pH $7.0 \pm 0.2$ and introduced onto a basic anion exchanger (5 ml LKB DEAE-Zetaprep/10mg heparin). After pre-elution with water, the main fraction was obtained by elution with 3 M sodium chloride solution. The collected fractions were precipitated by adding 3 volumes of 100% methanol. The precipitate was worked up and dried under vacuum.

Molecular weight 4800, 4400, 3900, 3500 (4 peaks in HPLC with respect to heparin standards).
Uronic acid content 96.4% (with respect to heparin).

## Example 4

### AT-III separation

The low-molecular (LM) heparin fraction obtained in Example 3 was introduced at 4 °C into a AT-III affinity column (10 mg LM-heparin/130 ml AT-III-MAAM-silica). The low-affinity fraction was obtained by elution with 1.5 bed volumes of 0.4 M sodium chloride solution in 0.05 M ammonium acetate at pH 7.5.

The low-affinity LM-heparin was collected at room temperature and introduced onto a basic ion exchanger. After washing with water, the ion exchanger was eluted with 3 M sodium chloride solution. The fractions collected were precipitated with 3 volumes of 100% methanol. The precipitate was worked up and dried under vacuum.

Molecular weight 4400, 3900 (2 peaks in HPLC with respect to heparin standards).
Uronic acid content 110.6% (with respect to heparin).

## Example 5

### Haemorrhagic activity

Capillary haemorrhaging test in rats.

Placebo or product to be tested was dosed via the dorsal vein of the penis of anaesthetized rats. After one minute a flap of skin was pulled loose by hand from the shaven abdomen along two previously made incisions. The wound was covered with a gauze bandage and the flap of skin was replaced over the gauze. After 10 minutes the gauze was removed and the blood contained therein extracted with 20 ml water. The haemoglobin concentration in the water was measured spectrophotometrically and used as a parameter for the blood loss. (See Thrombos.Haemostas. 42, 466, 1979).

| Substance | Dosage mg/kg | Haemorrhaging % |
|---|---|---|
| Placebo | – | 100 |
| Heparin | 8 | 222 |
| Product from ex 1 | 8 | 111 |
| Product from ex 3 | 5 | 147 |
| Product from ex 3 | 25 | 135 |

## Example 6

### Anti-HIV activity

Determination of the antiviral activity of the substances against HIV replication was carried out by means of inhibition of virus-induced cytopathogenicity, such as is measured with the 3'-(4,5-dimethylthiazol-2-yl)-2,5-diphenyltetrazolium bromide (MTT) method which is described by Pauwels et al. (J. Virol. Methods, 20, 1988), 309-321). With this method MT-4 cells are infected with HIV at 1000 $CCID_{50}$/ml.

After virus adsorption, the infected cells are washed and suspended in a culture medium. The number of MT-4 cells was brought to 3 x $10^5$ cells/ml. They were then introduced onto a plastic microtitre plate which contained various concentrations of the substance to be investigated. After incubating for 5 days at 37 °C the number of viable cells was determined the MTT-method. Anti-HIV-1 activity of the compounds was also determined by monitoring viral antigen expression in HUT-78 cells at day 12 after infection, using indirect immunofluorescence and laser flow cytofluorography as described by Baba et al. (Proc. Natl. Acad. Sci. USA, 85, (1988), 6132).

The 50% antiviral effective dosage ($ED_{50}$) on HIV-1 and HIV-2, the 50% cytotoxic dosage ($CD_{50}$) and the anti-thrombin activity are shown in the table.

| Substance | Anti-thrombin act. (E/mg) | HIV-1 (ED$_{50}$) ($\mu$g/ml) | HIV-2 (ED$_{50}$) ($\mu$g/ml) | CD$_{50}$ ($\mu$g/ml) |
|---|---|---|---|---|
| Heparin | 170 | 0.4 | 17.0 | >500 |
| Product from ex 1 | 0.6 | 0.2 | 1.3 | >500 |
| Product from ex 2 | 0.5 | 35.0 | 1.0 | >625 |
| Product from ex 3 | 39 | 2.5 | 2.0 | >625 |
| Product from ex 4 | 17 | 1.8 | N.T. | >625 |

N.T. not tested

## Claims

1. A use of heparin fractions or heparin fragments which are obtainable by periodate, heparinase, or nitrite degradation, having an antithrombin activity of less than 50 U/mg, for the manufacture of a medicament which inhibits or prevents the replication of the human immunodeficiency virus (HIV).

2. The use of heparin fractions or heparin fragments which are obtainable by periodate, heparinase, or nitrite degradation, having an antithrombin activity of less than 50 U/mg, for the manufacture of a medicament which inhibits or prevents the replication of HIV-1 and/or HIV-2.

3. The use of heparin fractions or heparin fragments according to claim 1 or 2, characterized in that their anti-thrombin activity is less than 20 U/mg.

4. The use of heparin fractions or heparin fragments according to claim 1 or 2, characterized in that their anti-thrombin activity is less than 1 U/mg.

5. The use of heparin fragments according to any one of the claims 1-4, wherein the heparin fragments are separated with the aid of anti-thrombin III (AT III) bonded to a solid carrier.

6. The use of heparin fragments according to claims 5, wherein the heparin fragments are obtainable by degradation with periodate.

7. The use of heparin fragments according to claims 5, wherein the heparin fragments are obtainable by degradation with nitrite.

## Patentansprüche

1. Verwendung von Heparinfraktionen oder Heparinfragmenten, welche durch Abbau mit Periodat, Heparinase oder Nitrit erhältlich sind und eine Antithrombinaktivität von weniger als 50 Einheiten/mg aufweisen, für die Herstellung eines Arzneimittels, welches die Vermehrung des menschlichen Immunschwäche-Virus (HIV) inhibiert oder verhindert.

2. Verwendung von Heparinfraktionen oder Heparinfragmenten, welche durch Abbau mit Periodat, Heparinase oder Nitrit erhältlich sind und eine Antithrombinaktivität von weniger als 50 Einheiten/mg aufweisen, für die Herstellung eines Arzneimittels, welches die Vermehrung von HIV-1 und/oder HIV-2 inhibiert oder verhindert.

3. Verwendung von Heparinfraktionen oder Heparinfragmenten nach Anspruch 1 oder 2, dadurch gekennzeichnet, dass ihre Antithrombinaktivität weniger als 20 Einheiten/mg beträgt.

4. Verwendung von Heparinfraktionen oder Heparinfragmenten nach Anspruch 1 oder 2, dadurch gekennzeichnet, dass ihre Antithrombinaktivität weniger als 1 Einheit/mg beträgt.

**5.** Verwendung von Heparinfragmenten nach einem der Ansprüche 1 bis 4, dadurch gekennzeichnet, dass die Heparinfragmenten mit Hilfe von Antithrombin III (AT III) gebunden an einen Träger abgetrennt werden.

**6.** Verwendung von Heparinfragmenten nach Anspruch 5, dadurch gekennzeichnet, dass die Heparinfragmentendurch Abbau mit Periodat erhältlich sind.

**7.** Verwendung von Heparinfragmenten nach Anspruch 5, dadurch gekennzeichnet, dass die Heparinfragmenten durch Abbau mit Nitrit erhältlich sind.

**Revendications**

**1.** Utilisation de fractions d'héparine ou fragments d'héparine qui peuvent être obtenus par dégradation au periodate, à l'héparinase ou au nitrite, ayant une activité antithrombotique inférieure à 50 U/mg, pour la fabrication d'un médicament qui inhibe ou empêche la réplication du virus de l'immunodéficience humaine (VIH).

**2.** Utilisation de fractions d'héparine ou fragments d'héparine qui peuvent être obtenus par dégradation au periodate, à l'héparinase ou au nitrite, ayant une activité antithrombotique inférieure à 50 U/mg, pour la fabrication d'un médicament qui inhibe ou empêche la réplication de VIH-1 et/ou VIH-2.

**3.** Utilisation de fractions d'héparine ou fragments d'héparine selon la revendication 1 ou 2, caractérisée par le fait que leur activité antithrombotique est inférieure à 20 U/mg.

**4.** Utilisation de fractions d'héparine ou fragments d'héparine selon la revendication 1 ou 2, caractérisée par le fait que leur activité antithrombotigue est inférieure à 1 U/mg.

**5.** Utilisation de fragments d'héparine selon l'une quelconque des revendications 1 à 4, dans laquelle les fragments d'héparine sont séparés à l'aide d'antithrombine III (AT III) fixée à un support solide.

**6.** Utilisation de fragments d'héparine selon la revendication 5, dans laquelle les fragments d'héparine peuvent être obtenus par dégradation au periodate.

**7.** Utilisation de fragments d'héparine selon la revendication 5, dans laquelle les fragments d'héparine peuvent être obtenus par dégradation au nitrite.